# FASCICULE DE BREVET EUROPEEN

(11) **EP 3 014 161 B1**
(45) Date de publication et mention de la délivrance du brevet: **13.06.2018**
(21) Numéro de dépôt: 14739888.7
(22) Date de dépôt: 25.06.2014
(51) Int. Cl.: F16L 37/098, F16L 33/025, A61J 1/14, A61M 39/10

(54) **CONNECTEUR FLUIDIQUE AVEC VERROUILLAGE**
VERRIEGELNDER FLUIDVERBINDER
LOCKING FLUID CONNECTOR

(30) Priorité: 28.06.2013 FR 1356352
(43) Date de publication de la demande: 04.05.2016
(73) Titulaire: SARTORIUS STEDIM FMT SAS, 13400 Aubagne (FR)
(72) Inventeur: BLAKE, Florian, F-83400 Hyeres (FR); GIBELIN, Jérémy, F-83330 Le Beausset (FR)
(74) Mandataire: Derambure Conseil
(86) Numéro de dépôt international: PCT/FR2014/051601
(87) Numéro de publication internationale: WO 2014/207382

(56) Documents cités:
- EP-A2- 1 457 727
- WO-A1-2005/073613
- WO-A1-2007/013813
- DE-U1- 20 321 171
- US-B2- 8 029 024

## Description

L'invention concerne les connexions fluidiques, en particulier les connexions ou raccordements fluidiques permettant de raccorder une conduite fluide à une autre conduite ou à un récipient, dans le domaine des applications biopharmaceutiques.

Plus précisément, les conduites ou tubes utilisés dans le domaine biopharmaceutique sont des tubes souples, qui servent à transporter des substances biopharmaceutiques diverses, avec le plus souvent des précautions d'asepsie nécessaires. Dans les applications biopharmaceutiques, ce type de tube souple permet la circulation, le passage, la communication d'un fluide, tel qu'un fluide biopharmaceutique et peut être raccordé au moyen d'une connexion fluidique soit à un tube souple similaire soit à un récipient ou un contenant, qui peut être rigide ou souple lui aussi.

Ledit récipient ou contenant peut-être une poche souple ou semi rigide, une enceinte rigide, un filtre ou une cartouche filtrante ou encore tout autre appareil utilisé dans un ensemble biopharmaceutique.

Dans une réalisation typique, par exemple, les connexions fluidiques présentent un diamètre intérieur utile compris entre par exemple 4 millimètres et 30 millimètres.

Dans les raccordements fluides entre tubes, poches, enceintes, cartouches filtrantes, et autre dispositifs biopharmaceutiques, il est très fréquent d'utiliser des connexions dites rapides où l'on vient accoupler un connecteur de type mâle dans un connecteur de type femelle. Les connecteurs en question sont habituellement réalisés en matière plastique.

En vue d'assurer une bonne performance en terme d'étanchéité avec prise en compte des dispersions de fabrication et les diverses tolérances admises, il est habituel d'installer un ou plusieurs joints d'étanchéité entre la partie mâle et la partie femelle. En pratique, on utilise souvent un joint torique placé dans une gorge annulaire.

Il est plus facile de réaliser une telle gorge sur la partie mâle (gorge extérieure) que sur la partie femelle (gorge intérieure), et par conséquent on dispose avantageusement le joint torique dans une gorge annulaire de l'embout mâle.

Par ailleurs, il est avantageux de prévoir un verrouillage de la connexion entre le connecteur femelle connecteur mâle de manière à éviter un débranchement non désiré ou intempestif de la connexion mâle-femelle. On obtient classiquement ce verrouillage par des moyens de clipsage. En pratique, on préfère prévoir ces moyens de clipsage sous forme intégrée par coopération de formes entre des éléments du connecteur femelle et du connecteur mâle, comme ceci est enseigné du document US8029024. Les documents DE 203 21 171 U1 et EP 1 457 727 A2 divulguent des dispositifs de connexion fluidique avec des moyens de clipsage, respectivement.

Pour des raisons de facilité d'obtention des pièces plastiques concernées, on dispose souvent au moins une languette élastique dans la partie femelle, cette languette élastique s'écartant vers l'extérieur lors du passage de l'embout mâle vers la position de couplage ; la languette élastique revient ensuite vers une position de repos dans laquelle elle bute contre une surface de butée appartenant au connecteur mâle, dans la position d'accouplement.

De plus, cette configuration favorise la sécurisation du clipsage, car il faut alors écarter les pattes flexibles pour pouvoir déverrouiller la connexion, ce qui est plus difficile à réaliser que de resserrer les pattes flexibles vers le centre (ce qui serait le cas si les pattes flexibles étaient agencées sur le connecteur mâle).

Une autre contrainte réside dans l'exigence de compacité radiale de la solution de couplage mâle-femelle avec son dispositif de clipsage, en particulier il faut veiller à diminuer l'encombrement radial total pour un diamètre intérieur utile donné de la connexion fluide.

Cependant, dans la configuration précitée, lors du mouvement d'insertion du connecteur mâle dans le connecteur femelle, il arrive que l'extrémité libre de la patte flexible interfère avec la surface extérieure du joint d'étanchéité lorsque celui-ci avance ce qui peut provoquer une détérioration du joint par exemple par rayure.

Il existe par conséquent le besoin de proposer une amélioration destinée à pallier, au moins en partie, un des inconvénients précités de l'art antérieur connu.

Ci-après, il est divulgué un exposé de l'invention telle que caractérisée dans les revendications.

Selon un premier aspect, l'invention a pour objet un dispositif de connexion fluidique apte et destiné à raccorder une première paroi délimitant un premier espace fluidique, à une seconde paroi délimitant un second espace fluidique, dans un ensemble biopharmaceutique, de sorte à être apte et destiné à assurer une communication fluidique entre le premier espace fluidique et le second espace fluidique, comprenant :
- un premier connecteur, délimitant un premier passage creux, apte et destiné à être raccordé à la première paroi et en communication fluidique avec le premier espace fluidique, le premier connecteur étant de type mâle, le premier connecteur comprenant au moins un joint d'étanchéité et une collerette de clipsage, le joint d'étanchéité s'étendant radialement depuis l'axe jusqu'à une première distance radiale R1, cette première distance étant mesurée avant couplage lorsque le joint d'étanchéité est monté sur le premier connecteur,
- un second connecteur délimitant un second passage creux, apte et destiné à être raccordé à la seconde paroi et en communication fluidique avec le second espace fluidique, le second connecteur étant de type femelle,
le premier connecteur et le second connecteur étant aptes et destinés à être couplés mutuellement dans une position de couplage relative, selon un axe A,
le deuxième connecteur comprenant au moins une patte flexible avec au moins une extrémité libre sur laquelle vient buter la collerette de verrouillage en position de couplage, pour empêcher un retrait à partir de la position de couplage,
l'extrémité libre la languette flexible étant séparée de l'axe au repos par une deuxième distance radiale R2, telle que R2 > R1, à savoir la deuxième distance radiale R2 est supérieure à la première distance radiale R1.

Moyennant quoi on ne risque pas d'abimer le joint torique par la languette flexible lors de l'insertion, et on garantit ainsi une qualité optimale de la fonction étanchéité au moyen du joint torique.

Selon l'invention chacune des languettes flexibles présente une extrémité libre dirigée vers le second espace fluidique. Moyennant quoi, les languettes flexibles sont protégées, par la partie avant du second connecteur, d'un endommagement mécanique qui pourrait subvenir avant le couplage ou pendant l'opération de couplage.

Selon une réalisation, la languette flexible est circonscrite par rapport à l'axe entre la deuxième distance radiale R2 et une troisième distance radiale R3, laquelle vérifie la relation 1 < R3/R2 < 1,4.
Moyennant quoi l'encombrement radial du connecteur femelle reste très limité.

Selon une réalisation, la collerette de clipsage présente une extension radiale maximum notée R6 qui vérifie la relation 1<R6/R2<1,3.
Moyennant quoi l'encombrement radial du connecteur mâle reste très limité.

Selon une réalisation, on dispose deux pattes flexibles diamétralement opposées. On propose ainsi une solution équilibrée, symétrique et particulièrement simple.

Selon une réalisation, on dispose deux joints toriques entre l'extrémité avant du premier connecteur et la collerette ; de sorte que l'on renforce le niveau d'étanchéité obtenu.

Selon une réalisation, le joint d'étanchéité est un joint torique ou un joint à lèvre en matériau élastomère.
Moyennant quoi le joint peut être compressé en position d'accouplement pour procurer une bonne performance en étanchéité. De plus il peut se déformer pendant le mouvement d'insertion d'accouplement.

Selon une réalisation, chaque patte flexible est reliée à un organe de déverrouillage obtenu intégralement par moulage du deuxième connecteur, et qui comprend un élément de liaison relié à la patte flexible, une zone d'appui, une jambe radiale agencée entre l'élément de liaison et la zone d'appui, la zone d'appui étant destinée à être pressée radialement vers l'intérieur pour écarter radialement la patte flexible vers l'extérieur et libérer ainsi la collerette de verrouillage.
Cette disposition permet de fournir une possibilité de déverrouiller la languette de verrouillage et par conséquent de désaccoupler les premier et second connecteurs.

Selon une réalisation, l'organe de déverrouillage comprend un moyen de verrouillage secondaire de la zone d'appui, pour empêcher le déplacement de la zone d'appui radialement vers l'intérieur, pour rendre ainsi la connexion doublement verrouillée.

On obtient ainsi un verrou secondaire qui permet de sécuriser le verrouillage primaire. Avantageusement, l'effacement du verrouillage secondaire peut donner lieu à une détérioration qui restera visible comme indicateur de tentative de déconnexion.

Selon une réalisation, le moyen de verrouillage secondaire est un doigt radial qui peut être déplacé entre une position inactive et une position active.
Ce qui représente une solution simple pour le verrouillage secondaire.

Selon une réalisation, le moyen de verrouillage secondaire forme un témoin de tentative de déverrouillage et peut comprendre une zone fusible.
Moyennant quoi, toute tentative d'effacement du verrou secondaire provoquera la rupture de la zone fusible et sera un indicateur visible rémanent de la tentative de déverrouillage.

Selon une réalisation, le second connecteur comprend une interface auxiliaire additionnelle de type femelle formée symétriquement par rapport à la première interface de type femelle, ladite interface auxiliaire étant configurée pour recevoir un troisième connecteur de type mâle.
Moyennant quoi, le second connecteur forme un raccord femelle-femelle permettant de raccorder entre eux deux connecteurs de type mâle.

Selon une réalisation, la collerette comprend un ergot anti-rotation, qui présente une extension radiale plus importante que le reste de la collerette. Moyennant quoi on peut empêcher une rotation relative entre le premier et le second connecteur en position de couplage.

Selon une réalisation, le premier connecteur ou le second connecteur comprend en outre un identifiant de type code barre ou étiquette RFID ou code couleur. Moyennant quoi l'accès à des informations relatives à la poche souple et/ou au produit biopharmaceutique y contenu est aisé, en particulier pour contribuer à la fonction traçabilité.

Selon un deuxième aspect, l'invention a pour objet un ensemble biopharmaceutique comprenant un dispositif de connexion fluidique tel que décrit ci-dessus.

On décrit maintenant brièvement les figures des dessins.
La figure 1 est une vue éclatée d'un dispositif de connexion conforme à la présente invention.
La figure 2 est une vue en coupe axiale du dispositif de connexion de la figure 1, en position séparée.
La figure 3 est une coupe axiale du dispositif de connexion de la figure 1, en position accouplée.
Les figures 4, 5 et 6 sont analogues à la figure 3 et montrent des variantes du dispositif de connexion, illustrant un organe de déverrouillage et un verrouillage secondaire.
La figure 7 illustre une variante du dispositif de connexion permettant de raccorder de connecteur mâle au moyen d'un connecteur double femelle.
Les figures 8A et 8B illustrent une variante du dispositif de connexion avec une fonction anti rotation.
Ci-après un exposé détaillé d'un mode de réalisation de l'invention assorti d'exemples et de référence aux dessins.
Dans l'exemple illustré aux figures 1 à 3, il s'agit de raccorder un premier connecteur **1,** de type mâle à un second connecteur **2** de type femelle.
Dans l'exemple illustré ici, le premier connecteur 1 est destiné à être raccordé à un tube souple 11 qui peut être défini généralement comme une première paroi 11 délimitant un premier espace fluidique **71.**
Dans l'exemple illustré ici, le second connecteur 2 est destiné à être raccordé à une poche biopharmaceutique 12 qui forme un second espace fluidique **72.**
Le premier connecteur **1** et le second connecteur **2** sont réalisés en matière synthétique, plus précisément ils peuvent être obtenus par moulage d'une matière plastique, par exemple polypropylène, du polyéthylène, du polycarbonate, du polysulfone, polyamide, PBT, ABS, ou autre matière plastique analogue.
Le premier connecteur **1** comprend une interface de connexion de type mâle, de forme générale de révolution autour de l'axe A, avec une extrémité avant **18,** une première gorge annulaire **65** prévue pour recevoir un premier joint d'étanchéité **35,** en l'occurrence ici un joint torique en matière élastomère, et optionnellement une seconde gorge annulaire **66** prévue pour recevoir un second joint d'étanchéité **36,** en l'occurrence ici aussi un joint torique en matière élastomère.
Les deux joints d'étanchéité sont disposés l'un à la suite de l'autre selon la direction axiale A, mais dans des positions radiales identiques, entre l'extrémité avant 18 et une collerette sera vue ci-après.
Lorsque le joint d'étanchéité (respectivement les joints d'étanchéité) est en place dans la gorge il occupe une dimension radiale maximum notée **R1,** distance mesurée à partir de l'axe lorsque que le joint d'étanchéité est en place dans la gorge mais que le premier connecteur n'est pas encore inséré dans le second connecteur.
En arrière des joints d'étanchéité, l'interface mâle comprend en outre une collerette d'arrêt **14** aussi appelé collerette de clipsage **14,** qui présente une extension radiale maximum référencée **R6.**
De manière optionnelle, cette collerette 14 peut comprendre un épaulement annulaire **14a** qui s'étend entre une position radiale intermédiaire **R4** et l'extension radiale maximum **R6.**
Le diamètre intérieur de l'espace creux **81** délimité par le cylindre intérieur du premier connecteur est référencé par **D9.**

Le premier connecteur **1** comprend en outre une interface de raccordement avec le premier espace fluidique, que celui-ci soit un tube, une poche souple, une cartouche filtrante. Dans l'exemple représenté, il s'agit d'un tube souple **11** qui vient s'insérer sur un embout **9** du premier connecteur.

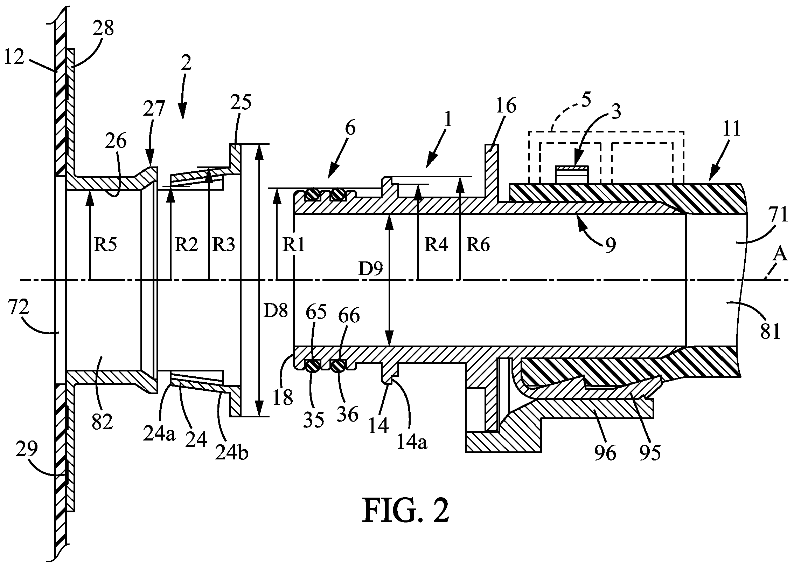

Deux variantes alternatives pour raccordement du tube 11 sur l'embout 9 sont illustrées la figure 2 :
- En partie supérieure de la coupe fig. 2, le tube 11 est serré sur l'embout par un collier de serrage classique **3** à oreille, protégé par un capot de protection **5** dessiné en ligne pointillée,
- En partie inférieure de la coupe fig. 2, le tube 11 est serré sur l'embout par des pattes flexibles intégrées **95** pressées radialement vers l'intérieur par une bague distincte **96** coulissante axialement.

Une collerette auxiliaire **16,** de plus grande dimension que la première collerette **14** déjà évoquée peut fournir plusieurs fonctionnalités, comme l'arrêt de la bague coulissante **96** et optionnellement son clipsage, elle peut faire office d'éléments de préhension pour un utilisateur, elle peut servir de butée d'arrêt pour l'insertion du tube. En outre cette collerette auxiliaire **16** peut servir de butée d'arrêt aux mouvements de couplage comme il sera vu ci-après.

Le second connecteur **2** comprend un cylindre intérieur **26** pour recevoir la partie mâle, de révolution autour de l'axe A et ayant un rayon référencé **R5.** Dans la position accouplée, le joint d'étanchéité **35** (respectivement 36) vient porter avec une compression radiale sur ce cylindre intérieur **26,** lequel forme un espace creux **82.** Par conséquent, on remarque que pour obtenir cette pression radiale, le rayon **R1** doit être légèrement supérieur au rayon **R5.**

En outre, le second connecteur **2** comprend au moins une languette flexible **24** prévue pour coopérer avec la collerette **14** susmentionnée afin de procurer le verrouillage de la position d'accouplement ; en l'occurrence ici on prévoit deux languettes flexibles diamétralement opposées, chacune ayant une emplanture **24b** et une extrémité libre **24a,** dirigée vers le second espace fluidique, c'est-à-dire vers l'arrière du second connecteur.

Plus précisément, pendant le mouvement d'insertion de la partie mâle, la collerette **14** écarte radialement vers l'extérieur les extrémités libres **24a** de chaque languette flexible. Dès que l'insertion du connecteur mâle a atteint une certaine course, la collerette **14** a dépassé les extrémités libres **24a** des languettes, et ces dernières reviennent vers l'intérieur par effet d'élasticité. Dans cette position, l'extrémité libre des languettes bute contre l'épaulement **14a** de la collerette **14,** ce qui empêche un retrait de la partie mâle ; on obtient ainsi un verrouillage de la position d'accouplement par clipsage. Toute autre coopération de formes entre des formes appartenant à la partie mâle et des formes appartenant à la partie femelle pourrait aussi être réalisée pour obtenir un résultat similaire.

Le second connecteur **2** comprend également disque de fixation **28,** prévu pour être fixé par soudure **29** à poche enveloppe **12** encore un filtre de produit biopharmaceutique. Toutefois le second connecteur **2** pourrait aussi être prévu pour être raccordé à un second tube souple de manière similaire à ce qui est présenté pour le premier connecteur.

Selon l'invention, l'extrémité libre **24a** de la languette flexible est séparée de l'axe par une deuxième distance radiale notée **R2,** telle que **R2 > R1,** à savoir la deuxième distance radiale R2 est supérieure à la première distance radiale R1. Ainsi, pendant le mouvement d'insertion, l'extrémité libre de la languette, même si elle présente une arête agressive ne peut pas blesser les joints toriques.

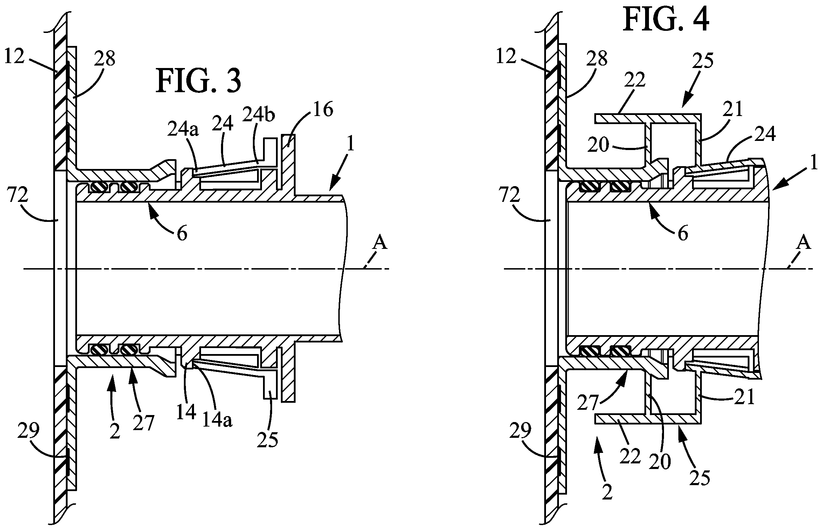

Selon un aspect avantageux, la languette flexible occupe radialement un espace limité ; en effet la languette flexible est circonscrite par rapport à l'axe entre la deuxième distance radiale **R2** et une troisième distance radiale **R3,** laquelle vérifie la relation **1** < **R3/R2** < **1,4.**
On obtient ainsi une solution particulièrement compacte si on compare cette troisième distance radiale **R3** au diamètre utile **D9** de la connexion fluidique.
Il faut noter ici que la distance radiale **R4** délimite l'épaulement **14a** sur lequel s'appuie l'extrémité des langues flexibles après le passage de la collerette de clipsage ; cette distance radiale **R4** est supérieure à la deuxième distance radiale **R2** de sorte que la languette flexible ne revient pas tout à fait à sa position de repos et conserve un effort radial résiduel dirigé vers l'intérieur (Fig. 3). Ceci permet de prendre en compte les dispersions de fabrication, les dispersions en comportement suivant les conditions d'environnement telles que l'humidité, la température, etc.
S'agissant de la collerette clipsage **14** du premier connecteur mâle, celle-ci présente une extension radiale maximum notée **R6** qui vérifie la relation 1<R6/R2<1,3. L'interface de couplage mâle **6** est ainsi réalisée de façon très compacte radialement. Il faut noter ici que la collerette auxiliaire 16 pourrait être absente ou de diamètre très inférieur par exemple similaire à celui de la collerette 14.

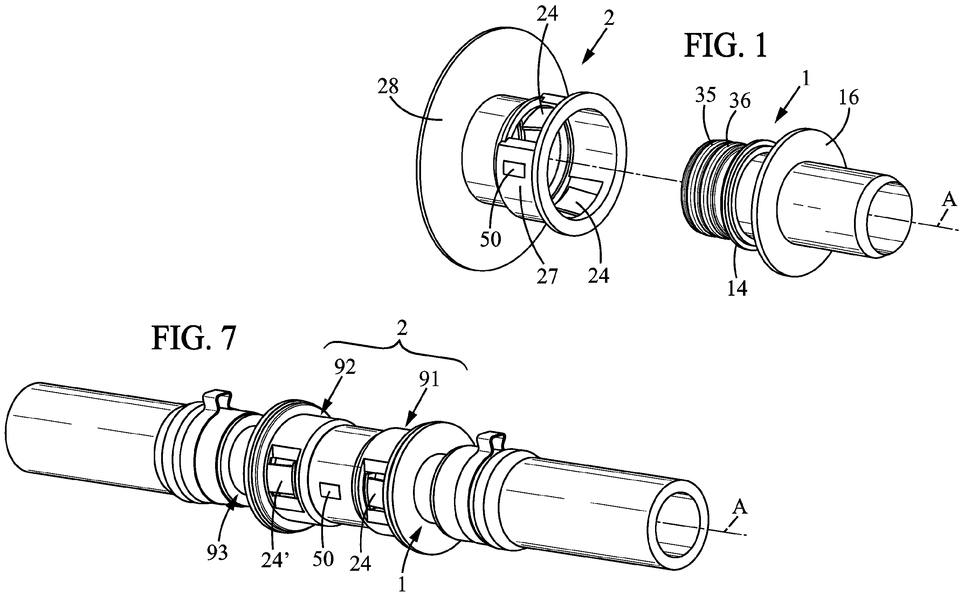

Dans l'exemple les figures 1 à 3, les languettes de verrouillage **24** s'écartent vers l'extérieur au moment où la deuxième collerette **14** avance au niveau de leur extrémité libre, puis reviennent vers l'intérieur pour se positionner sur l'arrière de la deuxième collerette 14. Après quoi il n'est pas possible de faire marche arrière c'est-à-dire de retirer le premier connecteur du second connecteur sans au préalable écarter d'une façon ou d'une autre les languettes de verrouillage 24, ce qui dans la pratique est impossible à réaliser manuellement et très difficile à réaliser avec un outil.

Cependant, dans la variante de réalisation illustrée à la figure 4, il est prévu sur chaque languette flexible **24** un organe de déverrouillage **25** obtenu intégralement par moulage du deuxième connecteur.
Cet organe de déverrouillage **25** comprend un élément de liaison **21** relié à la patte flexible, une zone d'appui **22,** une jambe radiale **20** agencée entre l'élément de liaison et la zone d'appui.
La zone d'appui **22** est destinée à être pressée radialement vers l'intérieur par exemple par les doigts d'un opérateur pour, moyennant un effet de pivot procuré par la jambe radiale **20,** écarter radialement la patte flexible **24** vers l'extérieur et libérer ainsi la collerette de verrouillage 14.
Dans la variante représentée à la figure 4, la zone d'appui est directement accessible et peut être manoeuvrée sans condition.

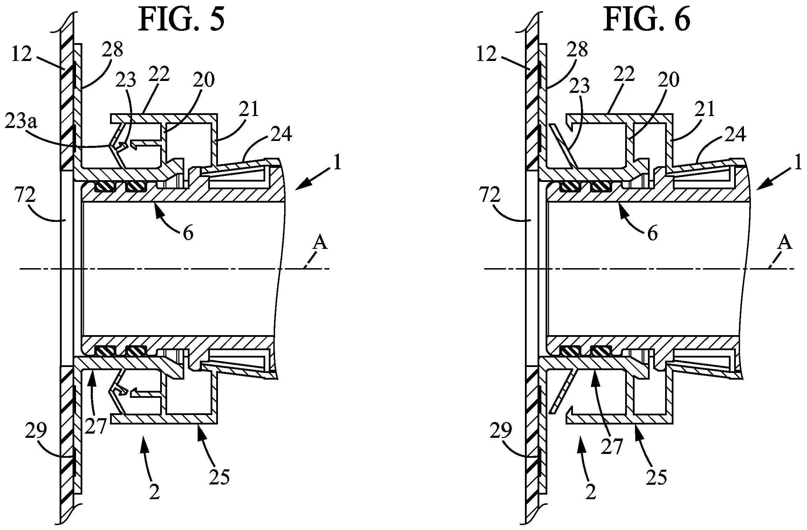

En revanche, les dans le cas des variantes représentées aux figures 5 et 6, on dispose un moyen de verrouillage secondaire **23** agencé entre la zone d'appui **22** et le corps **27** du second connecteur.

Selon la variante de la figure 6, le verrouillage secondaire 23 se présente comme un doigt radial qui peut être déplacé entre une position inactive (tel qu'illustré) et une position active ou il est déplacé vers la droite et se retrouve sous la zone d'appui 22 pour former un étai qui empêche la manoeuvre de pressions sur la zone d'appui 22. Si l'on veut procéder à la déconnexion de la connexion fluidique, il faut tout d'abord effacer le verrou secondaire 23 en déplaçant vers la gauche le doigt radial puis on procède à une pression radiale vers l'intérieur sur les zones d'appui 22, moyennant quoi on peut retirer le premier connecteur du second connecteur.

Selon la variante de la figure 5, un doigt radial comportant en outre un crochet qui rend plus difficile l'effacement du verrou secondaire. En outre, lorsque on efface le verrou secondaire, ceci entraîne la détérioration du doigt radial et génère une indication rémanente **23a** que la zone d'appui a été manoeuvrée, voire la rupture d'une zone fusible (non représentée).

Il faut remarquer que toutes les variantes du second connecteur présenté ci-dessus partagent une base commune ; l'organe de déverrouillage 25 peut être intégré optionnellement en fonction de la configuration du moule de formage. Il en est de même pour les variantes de la fonction verrouillage secondaire.

La figure 7 montre un autre mode de réalisation dans lequel le second connecteur **2** comprend deux interfaces de couplage femelle agencées tête-bêche le long d'un axe A, on peut ainsi appeler ce connecteur un raccord « double femelle ».
La première interface femelle **91** est destinée à recevoir par accouplement le premier connecteur mâle **1.** La seconde interface femelle **92** est destinée à recevoir par accouplement un troisième connecteur **93** de type mâle. Les interfaces mâle et femelle proprement dites sont identiques ou similaires à ce qui a été décrit ci-dessus. En particulier, la géométrie et la position des languettes de verrouillage 24,24' sont conformes à ce qui a été décrit ci-dessus pour éviter une blessure du joint torique par l'extrémité de ces languettes de verrouillage. Bien que non représentés, des organes de déverrouillage et optionnellement des verrous secondaires tels que décrit ci-dessus peuvent aussi équiper le connecteur double femelle **2.**

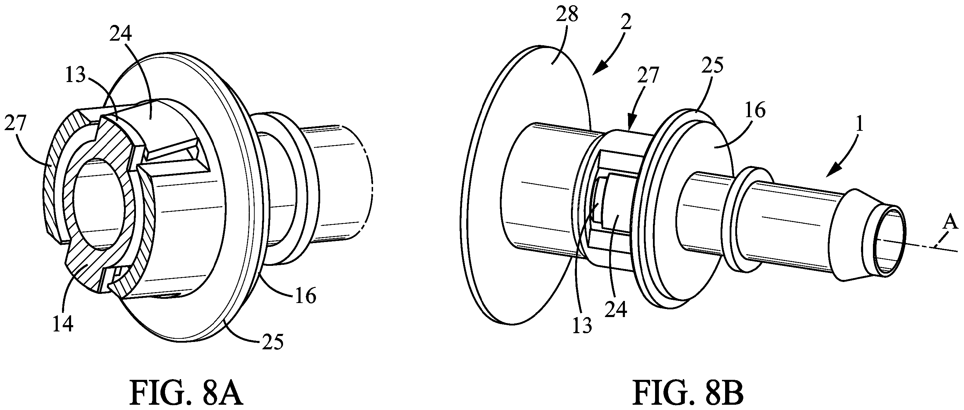

Les figures 8A et 8B représentent une variante de réalisation, dans lequel une fonction anti rotation est ajoutée. Plus précisément, la collerette de clipsage 14 se réduit à deux ergots **13** s'étendant radialement vers l'extérieur, chaque ergot étant prévu pour coopérer avec la languette flexible 24 de la même manière indiquée précédemment pour la collerette 14 de révolution.
Étant donné que les ergots **13** font saillie un peu plus vers l'extérieur, il forme une butée tangentielle vis-à-vis du corps **27** du connecteur ; ainsi un mouvement de rotation relatif du premier connecteur par rapport au second connecteur est empêché.

En outre, il faut remarquer la compacité axiale de l'accouplement mâle femelle avec fonction verrouillage intégrée. En effet, la distance axiale entre la collerette de clipsage **14** et l'extrémité avant **18** du connecteur mâle est inférieure au diamètre intérieur **D9** de la conduite utile. En outre, la distance axiale entre le disque de fixation **28** et l'emplanture des pattes flexibles **24b** et inférieure au double diamètre intérieur **D9** de la conduite utile. Par conséquent, à la fois le connecteur mâle 1 et le connecteur femelle 2 présentent une compacité axiale optimisée.

En outre, il est prévu une caractéristique optionnelle compatible avec toutes les variantes de réalisation précédemment évoquées : il s'agit de l'intégration d'un identifiant 50, de type code barre ou étiquette électronique par exemple de type RFID. De façon préférée, on dispose cet identifiant en surface sur le second connecteur 2, mais on pourrait dans le cas de l'étiquette RFID prévoir que celle-ci est disposée n'importe où sur le premier connecteur 1 et le second connecteur 2, accessible par lecture de type transpondeur.

## Revendications

1. Dispositif de connexion fluidique apte et destiné à raccorder une première paroi délimitant un premier espace fluidique (71), à une seconde paroi délimitant un second espace fluidique (72), dans un ensemble biopharmaceutique, de sorte à être apte et destiné à assurer une communication fluidique entre le premier espace fluidique et le second espace fluidique, comprenant :
- un premier connecteur (1), délimitant un premier passage creux (81), apte et destiné à être raccordé à la première paroi et en communication fluidique avec le premier espace fluidique, le premier connecteur étant de type mâle, le premier connecteur comprenant au moins un joint d'étanchéité et une collerette de clipsage (14), le joint d'étanchéité s'étendant radialement depuis l'axe jusqu'à une première distance radiale R1, cette première distance étant mesurée avant couplage lorsque le joint d'étanchéité est monté sur le premier connecteur,
- un second connecteur (2) délimitant un second passage creux (82), apte et destiné à être raccordé à la seconde paroi et en communication fluidique avec le second espace fluidique, le second connecteur étant de type femelle,
le premier connecteur et le second connecteur étant aptes et destinés à être couplés mutuellement dans une position de couplage relative, selon un axe A le deuxième connecteur comprenant au moins une patte flexible (24) avec au moins une extrémité libre (24a) sur laquelle vient buter la collerette de verrouillage (14) en position de couplage, pour empêcher un retrait à partir de la position de couplage,
la partie la plus radialement intérieure de la languette flexible est séparée de l'axe au repos et avant insertion, par une deuxième distance radiale R2,
telle que R2 > R1, à savoir la deuxième distance radiale R2 est supérieure à la première distance radiale R1, **caractérisé en ce que**, dans ce dispositif de raccord, chacune des languettes flexibles présente une extrémité libre (24a) dirigée vers le second espace fluidique.

2. Dispositif de raccord selon la revendication 1, dans lequel la languette flexible est circonscrite par rapport à l'axe entre la deuxième distance radiale R2 et une troisième distance radiale R3, laquelle vérifie la relation 1 < R3/R2 < 1,4.

3. Dispositif selon l'une des revendications 1-2, dans lequel la collerette de clipsage (14) présente une extension radiale maximum notée R6 qui vérifie la relation 1 <R6/R2<1 ,3.

4. Dispositif selon l'une des revendications 1-3, comprenant deux pattes flexibles (24) diamétralement opposées.

5. Dispositif selon l'une des revendications 1-4, comprenant deux joints toriques (35,36) entre l'extrémité avant (18) du premier connecteur et la collerette (14).

6. Dispositif selon l'une des revendications 1-5, dans lequel le joint d'étanchéité est un joint torique ou un joint à lèvre en matériau élastomère.

7. Dispositif selon l'une des revendications 1-6, dans lequel chaque patte flexible (24) est reliée à un organe de déverrouillage (25) obtenu intégralement par moulage du deuxième connecteur, et qui comprend un élément de liaison (21) relié à la patte flexible, une zone d'appui (22), une jambe radiale (20) agencée entre l'élément de liaison et la zone d'appui, la zone d'appui étant destinée à être pressée radialement vers l'intérieur pour écarter radialement la patte flexible vers l'extérieur et libérer ainsi la collerette de verrouillage.

8. Dispositif selon la revendication 7, dans lequel l'organe de déverrouillage (25) comprend un moyen de verrouillage secondaire (23) de la zone d'appui (22), pour empêcher le déplacement de la zone d'appui (22) radialement vers l'intérieur, et rendre ainsi la connexion doublement verrouillée.

9. Dispositif selon la revendication 8, dans lequel le moyen de verrouillage secondaire (23) est un doigt radial qui peut être déplacé entre une position inactive et une position active.

10. Dispositif selon l'une des revendications 8-9, dans lequel le moyen de verrouillage secondaire (23) forme un témoin de tentative de déverrouillage et peut comprendre une zone fusible.

11. Dispositif selon l'une des revendications 1-10, dans lequel le second connecteur comprend une interface auxiliaire (92) additionnelle de type femelle formée symétriquement par rapport à une première interface de type femelle (91), ladite interface auxiliaire étant configurée pour recevoir un troisième connecteur de type mâle (93).

12. Dispositif selon l'une des revendications 1-11, dans lequel la collerette (14) comprend un **ergot anti-rotation** (13), qui présente une extension radiale plus importante que le reste de la collerette.

13. Dispositif selon l'une des revendications 1-12, dans lequel le premier connecteur ou le second connecteur comprend en outre un identifiant (50) de type code barre ou étiquette RFID ou code couleur.

14. Ensemble biopharmaceutique comprenant un dispositif de connexion fluidique selon l'une des revendications 1 à 13.

## Patentansprüche

1. Fluidverbindungsvorrichtung, die geeignet und bestimmt ist, um in einer biopharmazeutischen Anordnung eine erste Wand, die einen ersten Fluidraum (71) begrenzt, mit einer zweiten Wand, die einen zweiten Fluidraum (72) begrenzt, derart zu verbinden, um geeignet und bestimmt zu sein, eine Fluidverbindung zwischen dem ersten Fluidraum und dem zweiten Fluidraum sicherzustellen, aufweisend:
- einen ersten Verbinder (1), der einen ersten hohlen Durchlass (81) begrenzt und geeignet und bestimmt ist, um an die erste Wand und in Fluidverbindung mit dem ersten Fluidraum angeschlossen zu werden, wobei der erste Verbinder des männlichen Typs ist, wobei der erste Verbinder mindestens ein Dichtungselement und einen Schnappkragen (14) aufweist, wobei das Dichtungselement sich radial von der Achse zu einem ersten radialen Abstand R1 erstreckt, wobei dieser erste Abstand vor einer Kopplung gemessen ist, wenn das Dichtungselement an dem ersten Verbinder angebracht ist,
- einen zweiten Verbinder (2), der einen zweiten hohlen Durchlass (82) begrenzt und geeignet und bestimmt ist, um an die zweite Wand und in Fluidverbindung mit dem zweiten Fluidraum angeschlossen zu werden, wobei der zweite Verbinder des weiblichen Typs ist,
wobei der erste Verbinder und der zweite Verbinder geeignet und bestimmt sind, um in einer relativen Kopplungsposition gemäß einer Achse A miteinander gekoppelt zu werden,
der zweite Verbinder mindestens eine flexible Klaue (24) mit mindestens einem freien Ende (24a) aufweist, an welchem der Verriegelungskragen (14) in der Kopplungsposition zum Anschlag kommt, um ein Abziehen aus der Kopplungsposition zu verhindern, wobei in Ruhestellung und vor einem Einsetzen der radial innerste Teil der flexiblen Zunge in einem zweiten radialen Abstand R2 von der Achse getrennt ist, derart, dass R2 > R1, dass also der zweite radiale Abstand R2 größer als der erste radiale Abstand R1 ist,
**dadurch gekennzeichnet, dass** in dieser Anschlussvorrichtung jede der flexiblen Zungen ein zum zweiten Fluidraum hin gerichtetes freies Ende (24a) aufweist.

2. Vorrichtung nach Anspruch 1, in welcher die flexible Zunge zwischen dem zweiten radialen Abstand R2 und einem dritten radialen Abstand R3, der die Beziehung 1 < R3/R2 < 1,4 erfüllt, bezüglich der Achse einbeschrieben ist.

3. Vorrichtung nach einem der Ansprüche 1 bis 2, in welcher der Schnappkragen (14) eine maximale radiale Erstreckung, mit R6 bezeichnet, aufweist, die die Beziehung 1 < R6/R2 < 1,3 erfüllt.

4. Vorrichtung nach einem der Ansprüche 1 bis 3, aufweisend zwei sich diametral gegenüberliegende flexible Klauen (24).

5. Vorrichtung nach einem der Ansprüche 1 bis 4, aufweisend zwei O-Ring-Dichtungen (35, 36) zwischen dem vorderen Ende (18) des ersten Verbinders und dem Kragen (14).

6. Vorrichtung nach einem der Ansprüche 1 bis 5, in welcher das Dichtungselement eine O-Ring-Dichtung oder eine Lippendichtung aus Elastomermaterial ist.

7. Vorrichtung nach einem der Ansprüche 1 bis 6, in welcher jede flexible Klaue (24) mit einem Entriegelungselement (25) verbunden ist, das mittels Formverfahren in einem Stück mit dem zweiten Verbinder hergestellt ist und das ein mit der flexiblen Klaue verbundenes Verbindungselement (21), eine Stützzone (22) und ein zwischen dem Verbindungselement und der Stützzone angeordnetes radiales Bein (20) aufweist, wobei die Stützzone dazu bestimmt ist, um radial nach innen gepresst zu werden, um die flexible Klaue radial nach außen zu spreizen und auf diese Weise den Verriegelungskragen freizugeben.

8. Vorrichtung nach Anspruch 7, in welcher das Entriegelungselement (25) eine sekundäre Verriegelungseinrichtung (23) der Stützzone (22) aufweist, um eine Bewegung der Stützzone (22) radial nach innen zu verhindern und dadurch die Verbindung doppelt verriegelt zu machen.

9. Vorrichtung nach Anspruch 8, in welcher die sekundäre Verriegelungseinrichtung (23) ein radialer Finger ist, der zwischen einer inaktiven Position und einer aktiven Position bewegt werden kann.

10. Vorrichtung nach einem der Ansprüche 8 bis 9, in welcher die sekundäre Verriegelungseinrichtung (23) Zeuge eines Entriegelungsversuchs darstellt und eine schmelzbare Zone aufweisen kann.

11. Vorrichtung nach einem der Ansprüche 1 bis 10, in welcher der zweite Verbinder eine zusätzliche Hilfsanschlussfläche des weiblichen Typs (92) aufweist, die symmetrisch bezüglich einer ersten Anschlussfläche des weiblichen Typs (91) gebildet ist, wobei die Hilfsanschlussfläche konfiguriert ist, um einen dritten Verbinder des männlichen Typs (93) aufzunehmen.

12. Vorrichtung nach einem der Ansprüche 1 bis 11, in welcher der Kragen (14) einen Antirotation-Vorsprung (13) aufweist, der eine größere radiale Erstreckung hat als der Rest des Kragens.

13. Vorrichtung nach einem der Ansprüche 1 bis 12, in welcher der erste Verbinder oder der zweite Verbinder desweiteren einen Identifikator (50) des Typs Strichcode oder RFID-Etikett oder Farbcode aufweist.

14. Biopharmazeutische Anordnung, aufweisend eine Fluidverbindungsvorrichtung nach einem der Ansprüche 1 bis 13.

## Claims

1. Fluid-connection device adapted and intended for connecting a first wall defining a first fluid space (71) to a second wall defining a second fluid space (72), in a biopharmaceutical assembly, so as to ensure a fluid communication between the first fluid space and the second fluid space, comprising:
- a first connector (1), defining a first hollow passage (81), adapted and intended for connection to the first wall and in fluid communication with the first fluid space, the first connector being of the male type, the first connector comprising at least one seal and a snap-fitting collar (14), the seal extending radially from the axis to a first radial distance R1, this first distance being measured before coupling when the seal is mounted on the first connector,
- a second connector (2) defining a second hollow passage (82), adapted and intended for connection to the second wall and in fluid communication with the second fluid space, the second connector being of the female type,
the first connector and the second connector being adapted and intended to be coupled together in a relative coupling position, along an axis A,
the second connector comprising at least one flexible tab (24) with at least one free end (24a) against which the locking collar (14) abuts when in the coupling position, to prevent withdrawal from the coupling position,
the radially innermost portion of the flexible tongue being distanced from the axis, at rest and prior to insertion, by a second radial distance R2,
wherein R2 > R1, namely the second radial distance R2 is greater than the first radial distance R1,
**characterized in that**
each of the flexible tongues has a free end (24a) oriented toward the second fluid space.

2. Connector according to claim 1, wherein the flexible tongue is circumscribed, relative to the axis, between the second radial distance R2 and a third radial distance R3 which satisfies the relation 1 < R3/R2 < 1.4.

3. Device according to one of claims 1-2, wherein the snap-fitting collar (14) has a maximum radial extension, denoted R6, which satisfies the relation 1 < R6/R2 < 1.3.

4. Device according to one of claims 1-3, comprising two flexible tabs (24) that are diametrically opposed.

5. Device according to one of claims 1-4, comprising two O-rings (35,36) between the front end (18) of the first connector and the collar (14).

6. Device according to one of claims 1-5, wherein the seal is an O-ring or a lip seal of elastomeric material.

7. Device according to one of claims 1-6, wherein each flexible tab (24) is connected to an unlocking member (25) that is integrally obtained when molding the second connector, and which comprises a connecting member (21) joined to the flexible tab, a bearing area (22), a radial arm (20) arranged between the connecting member and the bearing area, the bearing area being intended to be pressed radially inward to urge the flexible tab radially outward and thereby release the locking collar.

8. Device according to claim 7, wherein the unlocking member (25) comprises a secondary locking means (23) for the bearing area (22), to prevent radially inward movement of the bearing area (22), thus doubly locking the connection.

9. Device according to claim 8, wherein the secondary locking means (23) is a radially-oriented finger which is movable between an inactive position and an active position.

10. Device according to one of claims 8-9, wherein the secondary locking means (23) forms an indicator of an unlocking attempt and may comprise a mechanical fuse region.

11. Device according to one of claims 1-10, wherein the second connector comprises an additional auxiliary interface (92) of the female type, formed symmetrically with respect to a first interface (91) of the female type, said auxiliary interface being configured to receive a third connector (93) of the male type.

12. Device according to one of claims 1-11, wherein the collar (14) comprises an **anti-rotation lug** (13) which extends radially beyond the rest of the collar.

13. Device according to one of claims 1-12, wherein the first connector or the second connector further comprises an identifier (50) such as a barcode or RFID tag or color code.

14. Biopharmaceutical assembly comprising a fluid-connection device according to one of claims 1 to 13.
